# EUROPEAN PATENT SPECIFICATION

(11) **EP 0 577 913 B1**
(45) Date of publication and mention of the grant of the patent: **18.06.1997**
(21) Application number: 92850297.0
(22) Date of filing: 16.12.1992
(51) Int. Cl.: A61K 9/00, A61K 31/535

(54) **Ophthalmic composition containing timolol hemihydrate**
Augenarzneimittel enthaltend Timolol Hemihydrat
Compositions ophtalmiques à base de hémihydrate de timolol

(30) Priority: 08.07.1992 US 910665
(43) Date of publication of application: 12.01.1994
(73) Proprietor: LEIRAS OY, 20210 Turku (FI)
(72) Inventor: Perälampi, Markku, 36200 Kangasala (FI); Lehmussaari, Kari, 33560 Tampere (FI); Oksala, Olli, 33240 Tampere (FI); Pohjala, Esko, 33720 Tampere (FI); Reunamäki, Timo, 33700 Tampere (FI); Ruohonen, Jarkko, 21410 Vanhalinna (FI)
(74) Representative: Grew, Eva Regina

(56) References cited:
- EP-A- 0 034 542
- EP-A- 0 186 071
- EP-A- 0 448 856
- WO-A-90/04592
- US-A- 4 195 085

## Description

The present invention relates generally to the field of pharmaceutical preparations, more particularly to ophthalmic compositions made from (S)-timolol base, i.e. (S)-l-[(l,l-dimethylethyl)amino]-3-[[4-(4-morpholinyl)-1,2,5-thiadiazol-3-yl]oxy]-2-propanol, in crystalline form, i.e. in the form of its hemihydrate, as well as to a method for the topical treatment of glaucoma and ocular hypertension by applying such compositions into the eye of a human or an animal in need of such treatment. An alternative nomenclature commonly used in the art to refer to timolol base is 3-morpholino-4-(3-tert-butylamino-2-hydroxypropoxy)-1,2,5-thiadiazole.

### Background of the Invention

β-Adrenergic blockers were first reported to be useful for the treatment of glaucoma in 1967. Since then a vast number of β-blockers have been investigated for ophthalmic use in treating eye disorders such as glaucoma, some of which, e.g. timolol, betaxolol, carteolol, metipranolol, befunolol and levobunolol, have reached clinical use as well. However, all of the compounds currently used for the treatment of humans are associated with a number of side effects, notably cardiovascular, respiratory, central nervous system and ocular side effects (see e.g. J. Clin. Pharmacol. 29 (1989) 97).

This is also true of (S)-timolol, which is most commonly used in its maleate salt form as a drug for the treatment of glaucoma. The (S)-timolol free base in itself is, however, a non-crystalline substance which is generally difficult to handle and to make into accurate dosage forms. Compositions containing, as the active antiglaucoma agent, only (S)-timolol maleate, which is a well-crystallizing substance, as well as their use, are described in the US-patent 4,195,085. In the said compositions timolol base itself without maleate anion is not employed. The presence of the maleate anion is a necessary component of the compositions, but as regards the activity of the preparation is completely superfluous and only constitutes an unnecessary load on the eye.

A further and well known disadvantage of ophthalmic solutions of timolol maleate salt is its sensitivity to light, which imposes strict requirements on the packaging and storage conditions for the maleate salt product, as well as on the carefulness of the patient in handling the product. Furthermore, the presence of the maleate anion may in some formulations have a negative influence on and even destroy the effect of ion-sensitive components.

Use of salt forms such as timolol maleate usually leads to the need of a large amount of additional ions which are delivered into the eye and may cause harmful effects.

### Summary of the Invention

Thus it is an object of the present invention to provide an ophthalmological preparation from a crystalline and sufficiently soluble form of (S)-timolol itself without the additional load and other negative effects of the hitherto used salt anions, and which delivers (S)-timolol to the eye compartment in a sustained and controlled manner with minimum systemic absorption and/or no systemic side effects.

Recently a crystalline form of the (S)-timolol base and its synthesis, namely the (S)-timolol base hemihydrate, was discovered and disclosed, see WO-publication 90/04592.

Based on the discovery of its advantageous handling characteristics, therapeutic use of the said (S)-timolol base hemihydrate, particularly topical administration, is discussed in the parent application as being preferred. A detailed discussion of ophthalmic uses for non-crystalline or non-specified forms of (S)-timolol base in various vehicles has recently been published in Int. J. Pharm., 81 (1992) 59.

According to the present invention it has now surprisingly been discovered that many of the disadvantages involved in the use on the one hand of the (S)-timolol base and on the other hand of the (S)-timolol maleate salt can be avoided by providing an ophthalmic composition containing crystalline (S)-timolol base hemihydrate as the active agent.

### Detailed Description of the Invention

According to the invention it is possible to provide a number of novel formulations for eye therapy from the said (S)-timolol base hemihydrate, which formulations have inherently better sustained and controlled effects than those of corresponding formulations made from the (S)-timolol maleate salt. In general, the chemical and physical characteristics of the said (S)-timolol base hemihydrate makes it very suitable for the preparation of ophthalmic compositions, and especially in compositions where the base form of (S)-timolol as the active agent without associated maleate is the form of choice, more particularly where the high lipophilicity of the (S)-timolol base hemihydrate is to be made use of.

An illustrative series of physicochemical and in vitro tests have been performed. Preclinical studies with selected conventional or non-conventional formulations of (S)-timolol base hemihydrate have revealed that, in addition to the better physico-chemical properties of the base hemihydrate, the formulations according to the invention are at least as well suited for eye therapy as the conventional formulations made with the (S)-timolol maleate salt. The said formulation is very effective in lowering intraocular pressure after topical application, both in the normal and the glaucomatous eye. In addition, formulations according to the invention showed good patient compliance.

Further, the compositions according to the invention show better stability to light than the known timolol maleate salt compositions. Thus, in addition to not having to be protected from light by the patient, which is a clear user's advantage, the light stability of the (S)-timolol base hemihydrate compositions makes it possible to exploit new alternatives in packaging technology for timolol containing products. As compared to timolol maleate, it is more suitable for use with vehicles and devices (such as a unit dose) where transparency of the packaging is necessary or desirable.

As the maleate (or any unnecessary salt load) is missing, the adjustment of pH in compositions containing (S)-timolol base hemihydrate is more easily accomplished, and particularly insofar as the use of an additional base is not necessary and not used, unless otherwise desired for another reason. In the compositions according to the invention, e.g. desired ion-sensitive components can also be used without adversely affecting their desirable properties.

An important aspect in connection with the invention is the fact that (S)-timolol base hemihydrate of established crystal structure can be formed in practically 100% pure form, i.e. the traces of the enantiomeric, less active (R)-form can be completely removed in a single crystallization step, as is disclosed in the application WO 90/04592. Thus it is possible to use, in the composition, a purer active agent than can be obtained by the manufacture of the maleate salt, the manner of manufacture of which inevitably leads to the end product containing a disadvantageous amount of the (R)-enantiomer unless it is prepared from a 100% pure (S)-timolol base, e.g. from (S)-timolol base hemihydrate, or alternatively unless the (R)-enantioner containing maleate salt is purified by an additional and tedious purification process. Thus the manufacture of the composition is simplified as well and there is no need to make the maleate salt from the base hemihydrate as the timolol base hemihydrate functions at least as well as the maleate salt for the intended purpose.

The present invention thus provides an ophthalmic composition for topical use containing a therapeutically effective amount of pure (S)-timolol base hemihydrate together with an ophthalmic carrier.

A further object of the present invention is to provide a use of (S)-timolol base hemihydrate for the preparation of an ophthalmic composition for topical use, especially for the treatment of glaucoma and ocular hypertension in humans and animals.

A further object of the invention is to provide a method for the preparation of an ophthalmic composition for topical application or administration to the eye, especially for the treatment of glaucoma and ocular hypertension, wherein a therapeutically effective amount of (S)-timolol base hemihydrate is combined with an acceptable and effective ophthalmic carrier.

According to an advantageous embodiment, the composition according to the invention is in unit-dosage form.

The compositions according to the invention are particularly suitable for topical use. Examples of such compositions, aqueous or non-aqueous, are solutions, including oils and gels, suspensions, ointments and solid soluble or insoluble ocular inserts and other acceptable drug delivery systems known as such in the art. The concentration of active agent in the composition may vary but lies generally in the range of about 0.01 to to about 5, preferably between about 0.1 and about 2 percent by weight of the total composition. Higher doses such as for example up to about 10 percent by weight, or lower than those mentioned above, can be employed provided the dose is effective in lowering intraocular pressure. A unit dosage form contains typically an amount of 0.001 to about 5.0 mg, preferably about 0.005 to about 2 mg of active agent.

The compositions according to the invention contain at least an ophthalmologically suitable carrier known in the art. In the case of solutions and ointments, such a carrier may be water, a lower alkanol, polyols, polymeric alcohols, petrolatum, physiologically acceptable oils such as silicone oil, mineral oil, white oil and vegetable oils, for example ricin oil, peanut oil and other acceptable carriers, or any mixtures of two or more of the foregoing.

The solid inserts are made from a suitable polymer, such as acrylates, natural products, starch derivatives, other synthetic derivatives, and e.g. cellulose derivatives. A number of such products are commercially available in various molecular weight ranges, and are as such known in the art. Usually no enhancing agents for the liberation of the active substance are needed, as is the case with timolol maleate.

The eye inserts are typically made by mixing the active agent, together with optional additives, with the polymer in question in molten form, and forming of the mixture obtained into inserts of any suitable shape by moulding. An alternative route of manufacture comprises dissolving the components in a suitable solvent and then evaporating the solvent to form the insert, e.g. in the form of a film.

The compositions according to the invention may also contain selected ophthalmologically acceptable adjuvants admixed with the (S)-timolol hemihydrate active agent, for example (a) to adjust tonicity, such as sodium chloride, potassium chloride, glycerol, mannitol, sorbitol, sodium borate, sodium acetate and the like, (b) to adjust viscosity, such as cellulose derivatives, polyvinylpyrrolidone, polyvinyl alcohol, carboxyvinyl polymers and the like, (c) to adjust or to stabilize pH, such as conventional bases or acids or buffers, such as phosphate buffers, borate buffer or the like, (d) to increase solubility, and (e) to stabilize and to preserve the preparation, such as guaternary ammonium compounds, phenyl mercuric salts, thiomersal, methyl and propyl paraben, benzyl alcohol, phenylethanol and the like. Preparations without antimicrobial preservatives are provided in unit dose form.

The pH of the composition is suitably from 4 to 9.5 and most preferably from 6.5 to 7.5 and may be, as mentioned above regulated by adding a suitable buffer, such as a phosphate or borate buffer. For certain applications, the pH is advantageously adjusted by adding an acid, such as hydrochloric, acetic, boric, citric acid, and/or a base, such as an inorganic base, e.g. an alkali hydroxide, such as sodium hydroxide, or an organic base, such as a suitable amine. The pH of the compositions of solutions, and, where applicable, of suspensions, ointments and inserts, may thus be adjusted to higher or lower values depending on the nature of the carrier in order to improve the penetration of the active moiety through the liberation thereof from the carrier vehicle, or alternatively the ambient or unregulated pH of the carrier can be employed.

By means of the invention it is thus possible to make a wide range of various compositions, which may be optimized as regards their activity, by selectively choosing suitable carriers and/or adjuvants. The free (S)-timolol base hemihydrate gives wider possibilities for formulation without any limitations as compared to the maleate salt in which the presence and the role of the maleate component of the active agent has to be taken into consideration, such as when using ion-sensitive components.

The following describes non-limiting typical examples of compositions suitable for topical application to the eye for treating glaucoma and ocular hypertension, i.e. lowering intraocular pressure, according to the invention:

### Example 1 Solution

| Components | Multi dose | Unit dose |
|---|---|---|
| (S)-Timolol base hemihydrate | 2.56 mg | 2.56 mg |
| Benzalkonium chloride | 0.1 mg | - |
| Monosodium phosphate 2H₂0 | 10.53 mg | 10.53 mg |
| Disodium phosphate 2H₂0 | 12.01 mg | 12.01 mg |
| Water for injection q.s. | 1.0 ml | 1.0 ml |

The above components are admixed by dissolution in water. The resulting bulk solution is first sterilized by filtration and then filled aseptically into suitable containers.

### Example 2 Solution

| Components | Multi dose | Unit dose |
|---|---|---|
| (S)-Timolol base hemihydrate | 5.12 mg | 5.12 mg |
| Benzalkonium chloride | 0.1 mg | - |
| Monosodium phosphate 2H₂0 | 11.58 mg | 11.58 mg |
| Disodium phosphate 2H₂0 | 10.81 mg | 10.81 mg |
| Water for injection q.s. ad | 1.0 ml | 1.0 ml |

The above components are admixed by dissolution in water. The resulting bulk solution is first sterilized by filtration and then filled aseptically into suitable containers.

### Example 3 Solution

An eye drop formulation was prepared by combining the following ingredients in the amounts indicated.

| | |
|---|---|
| (S)-Timolol base hemihydrate | 0.2 % |
| Mannitol | 5.4 % |
| Water for injection q.s. | 1.0 ml |

The pH of this formulation may be adjusted to e.g. pH 7 by adding a suitable acid, such as hydrochloric acid.

### Example 4 Gel

| Components | Multi dose | Unit dose |
|---|---|---|
| (S) Timolol base hemihydrate | 2.56 mg | 2.56 mg |
| Benzalkonium chloride | 0.1 mg | - |
| Methylcellulose 4000 | 15.0 mg | 15.0 mg |
| Monosodium phosphate 2H₂0 | 10.53 mg | 10.53 mg |
| Disodium phosphate 2H₂0 | 12.01 mg | 12.01 mg |
| Water for injection q.s. ad | 1.0 ml | 1.0 ml |

The methylcellulose is dissolved in a portion of the sterile water and sterilized by autoclaving. All the other constituents are dissolved in a remaining portion of the water and sterilized by filtration. The water solution containing the other constituents is combined aseptically with the methyl cellulose gel.

### Example 5 Oil

| | |
|---|---|
| (S)-timolol base hemihydrate | 2.56 mg |
| Ricin oil q.s. ad | 1 ml |

The (S)-timolol base hemihydrate is dissolved in the ricin oil.

### Example 6 Ointment

| | |
|---|---|
| (S)-Timolol base hemihydrate | 2.56 mg |
| Petrolatum q.s. ad | 1.0 mg |

The above listed components are aseptically combined.

### Example 7 Insert

| | |
|---|---|
| (S)-Timolol base hemihydrate | 1 mg |
| Hydroxypropylcellulose q.s. ad | 12 mg |

The above components are molded together in a known manner to form eye inserts.

## Claims

1. An ophthalmic topical composition containing a therapeutically effective amount of (S)-(-)-3-morpholino-4-(3-tert-butylamino-2-hydroxypropoxy)-1,2,5-thiadiazole hemihydrate together with an ophthalmic carrier.

2. Ophthalmic composition according to claim 1 which is in the form of solution.

3. Ophthalmic composition according to claim 1 or 2 which is a unit dose form.

4. Ophthalmic composition according to claim 2 or 3 wherein the ophthalmic carrier is water.

5. Ophthalmic composition according to claim 1 which is in the form of a gel, an oil, an ointment or an insert.

6. Ophthalmic composition according to claim 1, wherein the therapeutically effective amount is an amount which effectively lowers the intraocular pressure.

7. Ophthalmic composition according to any one of the previous claims which contains 0.01 to 5% by weight, preferably 0.1 to 2 % by weight of (S)-(-)-3-morpholino-4-(3-tert-butylamino-2-hydroxypropoxy)-1,2,5-thiadiazole hemihydrate.

8. Ophthalmic composition according to claim 2 or 3 consisting of (S)-(-)-3-morpholino-4-(3-tert-butylamino-2-hydroxypropoxy)-1,2,5-thiadiazole hemihydrate, a water carrier and a buffer.

9. Ophthalmic composition according to claim 8 containing 0.001 mg to 5 mg of (S)-(-)-3-morpholino-4-(3-tert-butylamino-2-hydroxypropoxy)-1,2,5-thiadiazole hemihydrate.

10. Ophthalmic composition according to claim 1 further containing one or more adjuvants pharmaceutically acceptable for topical application to the eye, such as adjuvants are selected from the group consisting of tonicity adjustment agents, viscosity adjustment agents, pH adjustment agents, solubility adjustment agents and preservative agent, and liberation enhancing agents, where necesssary.

11. Ophthalmic composition according to claim 2 and 10, wherein the only anion present is that resulting from the pH-adjusting agent or the buffer, not including that of the optional viscosity enhancing agent and that of the optional antimicrobial agent.

12. Ophthalmic composition according to claim 2 or 3 and 10 wherein the adjuvants are comprised of non-ionic adjuvants, such as tonicity enhancing agent.

13. Use of (S)-(-)-3-morpholino-4-(3-tert-butylamino-2-hydroxypropoxy)-1,2,5-thiadiazole hemihydrate for the preparation of an ophthalmic composition for topical use, especially for the treatment of glaucoma and ocular hypertension in humans and animals.

14. Process for the preparation of an ophthalmic composition for topical use, especially for the treatment of glaucoma and ocular hypertension, wherein a therapeutically effective amount, preferably an intraocular pressure lowering amount of (S)-(-)-3-morpholino-4-(3-tertbutyl-amino-2-hydroxypropoxy)-1,2,5-thiadiazole hemihydrate is combined with an ophthalmic carrier.

15. Process of claim 14 further comprising admixing one or more selected pharmaceutically acceptable adjuvants together with the composition, such as adjuvants selected from the group consisting of tonicity adjustment agents, viscosity adjustment agents, pH adjustment agents, solubility adjustment agents and preservative agents, and liberation enhancing agents, where necessary.

16. Process of claim 14 or 15 wherein the composition is formed into a form selected from the group consisting of solutions, gels, oils, ointments and inserts.

17. Process of claim 14 or 16 wherein the composition is formed into a unit dosage form, preferably in the form of a solution.

18. Process of claim 17 wherein the composition is packaged in a transparent container.

## Patentansprüche

1. Ophthalmische topische Zusammensetzung, die eine therapeutisch wirksame Menge (S)-(-)-3-Morpholino-4-(3-tert-butylamino-2-hydroxypropoxy)-1,2,5-thiadiazol-Hemihydrat zusammen mit einem ophthalmischen Träger enthält.

2. Ophthalmische Zusammensetzung nach Anspruch 1, die in Form einer Lösung vorliegt.

3. Ophthalmische Zusammensetzung nach Anspruch 1 oder 2, die in Form einer Einheitsdosis vorliegt.

4. Ophthalmische Zusammensetzung nach Anspruch 2 oder 3, die als ophthalmischen Träger Wasser enthält.

5. Ophthalmische Zusammensetzung nach Anspruch 1, die in Form eines Gels, eines Öls, einer Salbe oder eines Inserts vorliegt.

6. Ophthalmische Zusammensetzung nach Anspruch 1, in der die therapeutisch wirksame Menge eine Menge ist, die den Augeninnendruck wirksam senkt.

7. Ophthalmische Zusammensetzung nach einem der vorangehenden Ansprüche, die 0,01 bis 5 Gew.-%, vorzugsweise 0,1 bis 2 Gew.-% (S)-(-)-3-Morpholino-4-(3-tert-butyl-amino-2-hydroxypropoxy)-1,2,5-thiadiazol-Hemihydrat enthält.

8. Ophthalmische Zusammensetzung nach Anspruch 2 oder 3, die aus (S)-(-)-3-Morpholino-4-(3-tert-butylamino-2-hydroxypropoxy)-1,2,5-thiadiazol-Hemihydrat, einem wäßrigen Träger und einem Puffer besteht.

9. Ophthalmische Zusammensetzung nach Anspruch 8, die 0,001 mg bis 5 mg (S)-(-)-3-Morpholino-4-(3-tert-butyl-amino-2-hydroxypropoxy)-1,2,5-thiadiazol-Hemihydrat enthält.

10. Ophthalmische Zusammensetzung nach Anspruch 1, die zusätzlich einen oder mehrere pharmazeutisch annehmbare Hilfsstoffe für die topische Anwendung im Auge enthält, ausgewählt aus der Gruppe bestehend aus tonizitätsregulierenden Stoffen, viskositätsregulierenden Stoffen, Stoffen zur Einstellung des pH-Wertes, löslichkeitsregulierenden Stoffen sowie Konservierungsmitteln und die Freisetzung erhöhenden Stoffen, falls erforderlich.

11. Ophthalmische Zusammensetzung nach Anspruch 2 und 10, in der das einzige anwesende Anion aus dem den pH-Wert einstellenden Stoff oder dem Puffer stammt, mit Ausnahme des aus einem gegebenenfalls vorhandenen viskositätserhöhenden Stoff und einem gegebenenfalls vorhandenen antimikrobiellen Stoff stammenden Anions.

12. Ophthalmische Zusammensetzung nach Anspruch 2 oder 3 und 10, in der die Hilfsstoffe aus nicht-ionogenen Hilfsstoffen, wie einem tonizitätserhöhenden Stoff, bestehen.

13. Verwendung von (S)-(-)-3-Morpholino-4-(3-tert-butyl-amino-2-hydroxypropoxy)-1,2,5-thiadiazol-Hemihydrat für die Herstellung einer ophthalmischen Zusammensetzung für die topische Anwendung, insbesondere zur Behandlung von Glaukom und erhöhtem Augeninnendruck bei Menschen und Tieren.

14. Verfahren zur Herstellung einer ophthalmischen Zusammensetzung für die topische Anwendung, insbesondere zur Behandlung von Glaukom und erhöhtem Augeninnendruck, bei dem man eine therapeutisch wirksame Menge, vorzugsweise eine den Augeninnendruck senkende Menge (S)-(-)-3-Morpholino-4-(3-tert-butylamino-2-hydroxypropoxy-1,2,5- thiadiazol-Hemihydrat mit einem ophthalmischen Träger kombiniert.

15. Verfahren nach Anspruch 14, bei dem man der Zusammensetzung zusätzlich einen oder mehrere ausgewählte pharmazeutisch annehmbare Hilfsstoffe, zum Beispiel ausgewählt aus der Gruppe bestehend aus tonizitätsregulierenden Stoffen, viskositätsregulierenden Stoffen, Stoffen zur Einstellung des pH-Wertes, löslichkeitsregulierenden Stoffen sowie Konservierungsmitteln und die Freisetzung erhöhenden Stoffen, falls erforderlich, zusetzt.

16. Verfahren nach Anspruch 14 oder 15, bei dem die Zusammensetzung zur Form einer Lösung, eines Gels, eines Öls, einer Salbe oder eines Inserts verarbeitet wird.

17. Verfahren nach Anspruch 15 oder 16, bei dem die Zusammensetzung zu einer Einheitsdosisform, die vorzugsweise in Form einer Lösung vorliegt, verarbeitet wird.

18. Verfahren nach Anspruch 17, bei dem die Zusammensetzung in einen transparenten Behälter verpackt wird.

## Revendications

1. Composition ophtalmique à usage topique contenant une quantité thérapeutiquement efficace d'hémihydrate de (S)-(-)-3-morpholino-4-(3-tert-butylamino-2-hydroxypropoxy)-1,2,5-thiadiazole conjointement avec un véhicule ophtalmique.

2. Composition ophtalmique selon la revendication 1 sous la forme d'une solution.

3. Composition ophtalmique selon la revendication 1 ou 2 sous la forme d'une dose unitaire.

4. Composition ophtalmique selon la revendication 2 ou 3, dans laquelle le véhicule ophtalmique est l'eau.

5. Composition ophtalmique selon la revendication 1 sous la forme d'un gel, d'une huile, d'un onguent ou d'un insert.

6. Composition ophtalmique selon la revendication 1, dans laquelle la quantité thérapeutiquement efficace est une quantité qui abaisse efficacement la pression intraoculaire.

7. Composition ophtalmique selon l'une quelconque des revendications précédentes, contenant de 0,01 à 5 % en poids, de préférence 0,1 à 2 % en poids, d'hémihydrate de (S)-(-)-3-morpholino-4-(3-tert-butylamino-2-hydroxypropoxy)-1,2,5-thiadiazole.

8. Composition ophtalmique selon la revendication 2 ou 3, constituée d'hémihydrate de (S)-(-)-3-morpholino-4-(3-tert-butylamino-2-hydroxypropoxy)-1,2,5-thiadiazole, d'un véhicule à base d'eau et d'un tampon.

9. Composition ophtalmique selon la revendication 8, contenant de 0,001 mg à 5 mg d'hémihydrate de (S)-(-)-3-morpholino-4-(3-tert-butylamino-2-hydroxypropoxy)-1,2,5-thiadiazole.

10. Composition ophtalmique selon la revendication 1, contenant, en outre, un ou plusieurs adjuvants pharmaceutiquement acceptables pour une application topique dans l'oeil, de tels adjuvants étant choisis dans l'ensemble constitué par les agents d'ajustement de la tonicité, les agents d'ajustement de la viscosité, les agents d'ajustement du pH, les agents d'ajustement de la solubilité et un agent de conservation, et les agents renforçant la libération, quand cela est nécessaire.

11. Composition ophtalmique selon les revendications 2 et 10, dans laquelle le seul anion présent est celui qui provient de l'agent d'ajustement du pH ou du tampon, ne comprenant pas celui qui provient de l'agent facultatif d'augmentation de la viscosité, ni celui provenant de l'agent antimicrobien facultatif.

12. Composition ophtalmique selon les revendications 2 ou 3 et 10, dans laquelle les adjuvants sont constitués d'adjuvants non ioniques, comme l'agent augmentant la tonicité.

13. Utilisation d'hémihydrate de (S)-(-)-3-morpholino-4-(3-tert-butylamino-2-hydroxypropoxy)-1,2,5-thiadiazole pour la préparation d'une composition ophtalmique à usage topique, en particulier pour le traitement d'un glaucome et de l'hypertension oculaire chez l'homme et chez l'animal.

14. Procédé de préparation d une composition ophtalmique à usage topique, en particulier pour le traitement d'un glaucome et de l'hypertension oculaire, dans lequel on combine une quantité thérapeutiquement efficace, de préférence une quantité abaissant la pression intra-oculaire, d'hémihydrate de (S)-(-)-3-morpholino-4-(3-tert-butylamino-2-hydroxypropoxy)-1,2,5-thiadiazole avec un véhicule ophtalmique.

15. Procédé selon la revendication 14, comprenant, en outre, l'étape consistant à mélanger un ou plusieurs adjuvants choisis, pharmaceutiquement acceptables, avec la composition, comme les adjuvants choisis dans l'ensemble constitué par les agents d'ajustement de la tonicité, les agents d'ajustement de la viscosité, les agents d'ajustement du pH, les agents d'ajustement de la solubilité et les agents de conservation, et des agents renforçant la libération, quand cela est nécessaire.

16. Procédé selon la revendication 14 ou 15, dans lequel la composition est transformée en un état choisi dans l'ensemble constitué par les solutions, les gels, les huiles, les onguents et les inserts.

17. Procédé selon la revendication 14 ou 16, dans lequel la composition est transformée en doses unitaires, de préférence sous la forme d'une solution.

18. Procédé selon la revendication 17, dans lequel la composition est emballée dans un conteneur transparent.
